# EUROPEAN PATENT APPLICATION

(11) **EP 2 404 598 A1**
(43) Date of publication of application: **11.01.2012**
(21) Application number: 10748772.0
(22) Date of filing: 03.03.2010
(51) Int. Cl.: A61K 31/198, A61K 9/14, A61K 31/401, A61K 31/405, A61K 31/4172, A61K 47/32, A61K 47/34, A61K 47/36, A61P 35/00

(54) **AMINO ACID-CONJUGATED CYANOACRYLATE POLYMER PARTICLES**

(30) Priority: 03.03.2009 JP 2009048756
(71) Applicant: PUBLIC UNIVERSITY CORPORATION YOKOHAMA CITY UNIVERSITY, Kanazawa-ku Yokohama-shi Kanagawa 236-0027 (JP)
(72) Inventor: SHIROTAKE, Shoichi, Yokohama-shi Kanagawa 236-0004 (JP); YOSHIDA, Atsushi, Yokohama-shi Kanagawa 236-0004 (JP); YOKOTA, Shumpei, Yokohama-shi Kanagawa 236-0004 (JP); ENDO, Itaru, Yokohama-shi Kanagawa 236-0004 (JP)
(74) Representative: Wills, Andrew Jonathan
(86) International application number: PCT/JP2010/053426
(87) International publication number: WO 2010/101178

(57) **Abstract**

Disclosed are cyanoacrylate polymer particles which comprise an amino acid(s) and have an average particle diameter of less than 1000 nm. The amino acid-containing particles according to the present invention can kill cancer cells by inducing apoptosis-like cell death. The particles have an especially high affinity for cell lines derived from lymphomas such as T-cell lymphoma and B-cell lymphoma. The particles can also exhibit an antiproliferative effect against some kinds of pancreatic cancer-derived cell lines. Therefore, the particles according to the present invention are useful for prevention and/or treatment of cancers.

## Description

### TECHNICAL FIELD

The present invention relates to cyanoacrylate polymer particles comprising an amino acid(s), and a therapeutic and/or prophylactic agent for a cancer(s) comprising the particles as an effective ingredient.

### BACKGROUND ART

T-cell lymphoma is one of the intractable non-Hodgkin lymphomas. There is no effective treatment other than myeloablative chemotherapy with a highly cytotoxic anticancer agent and subsequent bone marrow transplantation. Due to their strong cytotoxicity, conventional anticancer agents show not only the desired anticancer effect but also a strong toxic effect on normal cells. Therefore, normal cells in the patient are already damaged when the subsequent transplantation is performed, which often causes a poor prognosis. It is also not easy to find an appropriate donor because severe conditions must be cleared.

Pancreatic cancer is one of the cancers with the lowest survival rates. Since it does not have any specific early symptoms, pancreatic cancer is very difficult to detect at an earlier stage, and in many cases, is at a fairly advanced stage at the time of diagnosis. An effective therapy for pancreatic cancer has not been established yet, and its prognosis remains very poor.

On the other hand, in order to improve the effect of pharmaceuticals by drug delivery system (DDS) or by sustained release, studies of nano-encapsulation of drugs are now under way. For example, DDS in which a drug is encapsulated in cyanoacrylate polymer particles is known (Patent Documents 1 to 5 and Non-patent Document 1). The present inventors also have disclosed a method for producing cyanoacrylate polymer particles with little irregularity in particle diameter, antibiotic-containing particles, and plasmid-containing particles (Patent Documents 3 to 5). However, all the studies which have been carried out so far aimed at DDS or sustained release of drugs. Cyanoacrylate polymer particles which copmrise a substance having no special pharmacological actions in itself, such as an amino acid, are not known. Pharmaceuticals utilizing such particles are also not known.

### PRIOR ART DOCUMENTS

### PATENT DOCUMENTS

Patent Document 1: JP 11-503148 A
Patent Document 2: JP 2002-504526 A
Patent Document 3: JP 2008-127538 A
Patent Document 4: WO 2008/126846
Patent Document 5: JP 2008-208070 A

### NON-PATENT DOCUMENTS

Non-patent Document 1: Christine Vauthier et al., Adv. Drug Deliv. Rev., 55, 519-548 (2003)

### SUMMARY OF THE INVENTION

### PROBLEMS TO BE SOLVED BY THE INVENTION

Accordingly, an object of the present invention is to provide novel means useful for treatment of cancers.

### MEANS FOR SOLVING THE PROBLEMS

The present inventors intensively studied to synthesize nano-sized (i.e. an average particle diameter of less than 1000 nm), amino acid-containing cyanoacrylate polymer particles by anionically polymerizing cyanoacrylate monomers in the presence of an amino acid(s). They further studied to find that the amino acid-containing particles can kill various cells derived from cancer cells by inducing apoptosis-like cell death; that the particles are applicable to various kinds of cancers by selecting the kind of the amino acids to be contained therein; and that the particles can exhibit an especially high affinity for cells derived from lymphoma. They still further found that the particles have an antiproliferative effect against some kinds of pancreatic cancer-derived cell lines, and that hence, pancreatic cancers, for which no effective treatment is available, may also be treated with the particles. Thus the present invention has been completed.

That is, the present invention provides cyanoacrylate polymer particles which comprise an amino acid(s) and have an average particle diameter of less than 1000 nm. The present invention also provides a therapeutic and/or prophylactic agent for a cancer(s), comprising as an effective ingredient the particles according to the present invention above. The present invention further provides a method for treatment and/or prevention of a cancer(s), comprising administering an effective amount of the particles according to the present invention above to an animal in need of such treatment and/or prevention of a cancer(s).

### EFFECTS OF THE INVENTION

By the present invention, cyanoacrylate polymer particles comprising an amino acid(s) were first provided. The amino acid-containing particles can kill cancer cells by inducing apoptosis-like cell death, and therefore are useful for treatment and prevention of cancers. The affinity of the particles can be adjusted for various cancer cells such as epithelial or hematological cancer-derived cell lines by selecting the kind of the amino acids to be contained in the particles, and therefore are useful for treatment and prevention of various cancers including epithelial and hematological cancers. In particular, among cancer cells, the amino acid-containing particles have a high affinity for cells derived from T-cell lymphoma or B-cell lymphoma, which means that the particles are especially useful for treatment of lymphomas. The particles were also found to inhibit proliferation of some kinds of pancreatic cancer-derived cell lines. Therefore, it is expected that pancreatic cancers, for which no effective treatment is available, may also be treated with the particles. In clinical treatment of cancer, for example, the effect of the particles may be preliminarily determined by *in vitro* test on cancer cells separated from a patient to select amino acid-containing particles showing a high anticancer activity, and the selected particles may be administered to the patient, so that the cancer can be treated or prevented more effectively. That is, the therapeutic and/or prophylactic agent according to the present invention may be available as a tailor-made anticancer agent. Since the amino acid-containing particles of the present invention may be prepared using biocompatible materials, the particles are highly safe for human beings and very advantageous in the clinical use compared with the known anticancer agents. By the present invention, cancers can be treated without using highly cytotoxic anticancer agents nor DDS, and therefore the present invention can also contribute to the improvement of quality of life of the patients.

### BRIEF DESCRIPTION OF THE DRAWINGS

Fig. 1 shows the survival rate of various cell lines treated with Lys-containing particles.
Fig. 2 shows the survival rate of various cell lines treated with Arg-containing particles.
Fig. 3 shows the survival rate of various cell lines treated with His-containing particles.
Fig. 4 shows the survival rate of various cell lines treated with Asp-containing particles.
Fig. 5 shows the survival rate of various cell lines treated with Gly-containing particles.
Fig. 6 shows the survival rate of various cell lines treated with Ala-containing particles.
Fig. 7 shows the survival rate of various cell lines treated with Val-containing particles.
Fig. 8 shows the survival rate of various cell lines treated with Leu-containing particles.
Fig. 9 shows the survival rate of various cell lines treated with Try-containing particles.
Fig. 10 shows the survival rate of various cell lines treated with Phe-containing particles.
Fig. 11 shows the survival rate of various cell lines treated with Ser-containing particles.
Fig. 12 shows the survival rate of various cell lines treated with Thr-containing particles.
Fig. 13 shows the survival rate of various cell lines treated with Met-containing particles.
Fig. 14 shows the survival rate of various cell lines treated with particles containing no amino acid.
Fig. 15 shows the result of Annexin V assay on EL-4 cells treated with Arg-containing particles.
Fig. 16 shows the antiproliferative effect of various amino acid-containing particles against YCUB-2 cells derived from human B-cell lymphoma.
Fig. 17 shows the survival rate of YCUB-2 cells derived from human B-cell lymphoma after 48-hour treatment with amino acid (Arg, Asp)-containing particles at the concentrations indicated.
Fig. 18 shows the survival rate of H9 cells derived from human T-cell lymphoma after 24-hour treatment with amino acid (Arg, Asp)-containing particles at the concentrations indicated.
Fig. 19 shows a graph for comparison of antiproliferative actions against H9 cells between the known anticancer agents, mitomycin-C (MMC) and actinomycin-D (Act-D), and amino acid-containing particles.
Fig. 20 shows the survival rate of H9 cells derived from human T-cell lymphoma treated with Asp-containing particles for 1, 3, 6, or 24 hours at the concentrations indicated.
Fig. 21 shows the survival rate of H9 cells derived from human T-cell lymphoma treated with Arg-containing particles for 1, 3, 6, or 24 hours at the concentrations indicated.
Fig. 22 shows the survival rate of H9 cells derived from human T-cell lymphoma after 24-hour treatment with the anticancer agent mitomycin-C (MMC) or actinomycin-D (ACD) at various concentrations.
Fig. 23 shows the proliferation inhibition rate determined in H9 cells treated with three kinds of amino acid-containing particles (basic amino acid D70Arg-NP, acidic amino acid D70Asp-NP, neutral amino acid D70Gly-NP) or particles containing no amino acid (D70-NP).
Fig. 24 shows the antiproliferative action of amino acid-containing particles (D70Asp-NP, D70Arg-NP, D70Gly-NP), which was examined using various cell lines derived from human pancreatic cancer. D70-NP indicates particles containing no amino acid.

### MODE FOR CARRYING OUT THE INVENTION

The particles according to the present invention consist of cyanoacrylate polymer and comprise an amino acid(s). As for the particle size, they have an average particle diameter of less than 1000 nm. The lower limit of the particle size is not restricted. For example, if the polymer particles are produced by polymerization of acrylate monomers in the below-described manner, the particle diameter is typically not less than about 7 nm. The average particle diameter of the particles is preferably 20 nm to 600 nm, more preferably 50 nm to 550 nm. The electric charge (Zeta potential) of the particles is not restricted, and usually about -40 mV to 0 mV. "Zeta potential" represents an electric charge of the surface of particles, and is an indicator of the dispersion stability of particles. The particle size and the Zeta potential may be easily measured with, for example, a commercially available device utilizing a He-Ne laser (such as Zetasizer manufactured by Malvem Inst.UK).

The kind of the amino acid used is not restricted. Typically, any of the 20 kinds of amino acids which consist of natural proteins is(are) used. As described below, the kind of the amino acid contained in the particles of the present invention has an influence on the anticancer activity of the particles. The amino acid contained in the particles may be one amino acid, or may be two or more amino acids.

The cyanoacrylate polymer moiety of the above-described amino acid-containing particles may be obtained by anionically polymerizing cyanoacrylate monomers. The cyanoacrylate monomer used is preferably alkyl cyanoacrylate monomer (the alkyl group is preferably a C₁-C₈ alkyl). Especially preferred is *n-*butyl-2-cyanoacrylate (nBCA) represented by the following formula, which is conventionally used as an adhesive for wound closure in the field of surgery.

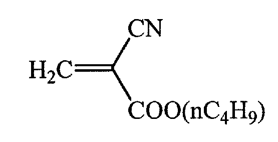

Although not restricted, in the above-mentioned anionic polymerization, a saccharide(s) and/or polysorbate(s) may be used for initiation and stabilization of the polymerization. Therefore, "cyanoacrylate polymer" as used herein includes those containing a polymerization initiator/stabilizer such as a saccharide(s) and/or polysorbate(s). It is known that, as disclosed in Patent Document 4, a polysorbate(s) can be used as a polymerization initiator/stabilizer in the polymerization reaction of cyanoacrylate polymer particles.

Saccharides are not restricted, and may be any of monosaccharides having a hydroxyl group(s), disaccharides having a hydroxyl group(s) and polysaccharides having a hydroxyl group(s). Examples of the monosaccharide include glucose, mannose, ribose, fructose and the like. Examples of the disaccharide include maltose, trehalose, lactose, sucrose and the like. Examples of the polysaccharide include dextran, which is used for polymerization of conventional cyanoacrylate polymer particles, mannan (see, Patent Document 5) and the like. These saccharides may be either in the cyclic form or in the chain form, and in case of cyclic form, saccharides may be either in the pyranose form or in the furanose form. Saccharides have various isomers, and any of such isomers may be used in the present invention. Usually, monosaccharides exist in the form of pyranose or furanose, and such monosaccharides are α- or β-linked to form a disaccharide. Saccharides in such an ordinary form may be used without modification.

Polysorbates are not restricted, and may be any of the known Tween series surfactants such as polyoxyethylene sorbitan monolaurate (trade name: Tween 20), polyoxyethylene sorbitan monooleate (trade name: Tween 80) and the like.

Monosaccharides, disaccharides and polysaccharides and polysorbates may be used individually, or two or more of these may be used in combination. Among the saccharides and polysorbates described above, glucose, dextran and Tween 20 (trade name) are preferred, and dextran is especially preferred. As for dextran, one having an average molecular weight of not less than 70,000 is preferred. The upper limit of the molecular weight of dextran is not restricted, and the molecular weight is usually not more than about 500,000.

The amino acid containing particles may be prepared by synthesizing polymer particles and then immersing the particles into an amino acid solution, or by carrying out the anionic polymerization of monomers in the presence of an amino acid(s). The latter is preferred because the amino acid(s) can be efficiently incorporated into the particles thereby. Particles containing two or more amino acids may be produced by carrying out anionic polymerization in the presence of the two or more amino acids.

As a solvent for the polymerization, water is typically used. Because the anionic polymerization is initiated by hydroxide ion, the polymerization velocity is influenced by pH of the reaction solution. When pH of the reaction solution is high, polymerization proceeds rapidly because of a high concentration of hydroxide ion. When pH is low, polymerization proceeds slowly. In preparation of amino acid-containing particles, an appropriate polymerization velocity is usually attained under an acidic condition of about pH1.5 to 3.0. The acid added to the reaction solution in order to acidify it is not restricted, and hydrochloric acid may be preferably used as it does not have a bad influence on the reaction and vaporizes after the reaction. The concentration of hydrochloric acid is not restricted, and may be about 0.0005 N to 0.5 N. The concentration of hydrochloric acid may be appropriately selected depending on the nature of the amino acid(s) used, for example, the concentration may be about 0.05 N when incorporating a basic amino acid(s), and may be about 0.01 N when incorporating a neutral and/or acidic amino acid(s).

The polymerization may be carried out by, for example, dissolving the amino acid(s) to be contained in the particles and the above-described polymerization initiator/stabilizer (i.e. a saccharide(s) and/or polysorbate(s)) in a solvent, adding thereto cyanoacrylate monomers under stirring, and preferably continuing stirring. The reaction temperature is not restricted, and the room temperature is preferred because of the simplicity. The reaction time may be appropriately selected depending on the pH of the reaction solution, the kind of the solvent, and the concentration of the polymerization initiator/stabilizer, because the reaction velocity varies depending on these factors. Although not restricted thereto, the reaction time is usually about 10 minutes to 4 hours, preferably about 30 minutes to 3 hours. Because the obtained amino acid-containing particles are usually used as neutral particles, it is preferred to add a base such as an aqueous sodium hydroxide solution to the reaction solution after completion of the reaction to neutralize it.

The concentration of cyanoacrylate monomers in the polymerization reaction solution at the beginning of the reaction is not restricted, and is usually about 0.5 v/v% to 2.0 v/v%, preferably about 0.8 v/v% to 1.2 v/v%. The concentration of the amino acid(s) in the polymerization reaction solution at the beginning of the reaction is not restricted, and is usually about 0.02 w/v% to 2 w/v%. In cases where a saccharide(s) and/or polysorbate(s) is(are) used in the polymerization reaction, the concentration of the saccharide(s) and/or polysorbate(s) (in cases where two or more are used, the total concentration thereof) in the polymerization reaction solution at the beginning of the reaction is not restricted, and is usually about 0.5% to 10%, preferably about 0.75% to 7.5%. As used herein, the concentration of the saccharide(s) means w/v%, and the concentration of the polysorbate(s) means v/v%. For example, in the case where a single saccharide is used, the concentration ranges described above mean "0.5 w/v% to 10 w/v%" and "0.75 w/v% to 7.5 w/v%", respectively. In the case where 5 w/v% of saccharide(s) and 1 v/v% of polysorbate(s) are used in combination, the total concentration thereof is expressed as 6%. It should be noted that, in the case where a monosaccharide(s) (e.g. glucose) is(are) used alone, the monosaccharide(s) is(are) preferably used at a concentration of about 2.5 w/v% to 10 w/v%.

By the above-described polymerization reaction, amino acid-containing nanoparticles having an average particle diameter of less than 1000 nm may be easily produced. The particle size can be regulated by regulating the cyanoacrylate monomer concentration in the reaction solution, pH, and/or the reaction time. In the case where a saccharide(s) and/or polysorbate(s) is(are) used as a polymerization initiator/stabilizer, the particle size can also be regulated by changing the concentration and/or the kind of the polymerization initiator/stabilizer (see, e.g. Patent Documents 3, 4). In general, the particle size becomes bigger in the case where the reaction solution has a higher pH, where the reaction is carried out for a longer time, or where the saccharide concentration in the reaction solution is lower; whereas, the particle size becomes smaller in the case where a polysorbate(s) is(are) used as a polymerization initiator/stabilizer. The particles having a desired size may be produced by appropriately combining these reaction conditions. For example, as shown in the Examples below, particles with an average particle diameter of about 120 nm to 500 nm can be obtained by carrying out the polymerization reaction for about 2 hours at a pH of about 2 using a polysaccharide(s) at a concentration of about 1 w/v% and a cyanoacrylate monomer at a concentration of about 1 v/v%. The amino acid incorporation ratio of the particles obtained by the above-described method is usually about 6% to 60%.

As shown in the Examples below, the above-described amino acid-containing particles have a cytotoxic activity against various cancer cells (such as cervical cancer, T-cell lymphoma, B-cell lymphoma, monocytic leukemia, renal cancer, pancreatic cancer). On the other hand, the particles are not cytotoxic to normal cells, since nothing unusual is found in the mice to which the particles are administered. Therefore, the amino acid-containing particles may exhibit a cytotoxic activity specifically to cancer cells existing in a living body when administered to the living body, and hence the particles are useful as a therapeutic and/or prophylactic agent for a cancer(s).

The cancer(s) to be treated with the therapeutic and/or prophylactic agent according to the present invention is(are) not restricted. The agent is applicable to various cancers including uterine cancers (such as cervical cancer), lymphomas (such as non-Hodgkin lymphomas including T-cell lymphoma, B-cell lymphoma, etc.), leukemias (such as monocytic leukemia), renal cancer, pancreatic cancer and the like. The kind of amino acid-containing particles showing the most effective anticancer action varies depending on the kind of cancers, and also may vary depending on the patient even against the same cancer. In the present invention, an amino acid(s) to be contained in particles may be appropriately selected to treat various cancers. Particles containing no amino acid can also inhibit proliferation of cancer cells when used at a high concentration, and the anticancer activity of particles can be remarkably improved by incorporating an amino acid(s) thereinto. In particular, especially remarkable elevation of the cytotoxic activity by incorporation of amino acids is observed against cells derived from lymphoma (see, Examples below). Therefore, the therapeutic and/or prophylactic according to the present invention is especially highly effective against lymphomas.

The fact that the anticancer activity of the amino acid-containing particles is affected by the kind of amino acid(s) contained in the particles is concretely demonstrated in the Examples below.

For example, most of the 20 amino acids confer a strong antiproliferative action against human B-cell lymphoma cells on the particles. Specifically, although arginine-containing particles cannot exhibit an anticancer activity when used at a concentration of 10 µg/ml or less, particles containing glycine, methionine, glutamic acid, aspartic acid, lysine, alanine, valine, serine, cysteine, phenylalanine, histidine, leucine, threonine, tryptophan, proline, asparagine or glutamine inhibit proliferation of cancer cells. Among these, glycine-, glutamic acid-, aspartic acid- or histidine-containing particles have a prominent anticancer activity.

As for human T-cell lymphoma cells, for example, arginine-containing particles and aspartic acid-containing particles have an antiproliferative effect against human T-cell lymphoma cells equal to or higher than known anticancer agents such as mitomycin-C, actinomycin-D and the like. Particles containing a neutral amino acid (glycine, alanine, valine, leucine, isoleucine, serine, threonine, cysteine, methionine, phenylalanine, tryptophan, tyrosine, proline) have an especially high antiproliferative effect, and among these, for example, glycine-containing particles have a remarkably high anticancer activity.

As for human pancreatic cancer cells, particles containing no amino acid also show some antiproliferative effect against human pancreatic cancer cells, and this antiproliferative effect is potentiated by amino acid incorporation against some of the cell lines. Therefore, the amino acid-containing particles may be effective means for treatment of some kinds of pancreatic cancers. Specifically, compared with particles containing no amino acid, aspartic acid-containing particles have a higher antiproliferative effect against human pancreatic cancer cell line MIA-PaCa2, and glycine- and aspartic acid-containing particles have a higher antiproliferative effect against AsPC-1. These amino acid-containing particles may be effective against pancreatic cancers which have the same characteristics (e.g., response to particular chemical substances, expression pattern of particular genes) as above-mentioned pancreatic cancer cell lines.

The data described in the following Examples demonstrates not only that particular amino acid-containing particles may be established as means for treating a particular cancer(s), but also that cancers may be treated more effectively by selecting the kind of amino acid(s) to be contained in particles depending on the kind of cancer and the nature of cancer in an individual patient. For example, against human B-cell lymphoma, particles containing glycine, glutamic acid, aspartic acid and/or histidine may be established as a therapeutic and/or prophylactic agent which can be used generally therefor. In the case where the kind of amino acid-containing particles is selected for each patient, for example, using a cancer cell sample collected from the cancer lesions in the patient, the anticancer activity (e.g. cytotoxic activity to cancer cells) of a series of amino acid-containing particles is examined to determine which particles exhibit a high anticancer activity. If amino acid-containing particles which exhibit an anticancer activity significantly higher than a known anticancer agent and/or control polymer particles containing no amino acid are found, the particles may be selected as particles to be administered to the above-mentioned patient. In cases where the particles are used as a therapeutic and/or prophylactic agent for a cancer(s), the particles may also comprise a single amino acid or two or more amino acids. In addition, a mixture of particles containing different amino acids may be used as a therapeutic and/or prophylactic agent. Therefore, if a plurality of kinds of amino acid-containing particles are determined to be effective in the *in vitro* test, all the determined amino acids may be incorporated into the same or different particles, and the prepared particles may be administered to the patient. Tailor-made medicine in which treatment is optimized for individual patients can be achieved by selecting amino acid-containing particles which are expected to have the highest effect in a patient and administering the selected particles to the patient. Various methods are known for evaluating an anticancer activity using cell samples, and any of such known methods may be used. For example, MTT assay described in Examples may be used.

Animals to be treated with the therapeutic and/or prophylactic agent for a cancer(s) are not restricted, and are preferably mammals such as humans, dogs, cats, rabbits, hamsters, monkeys, mice, horses, sheep, cows and the like. These animals to be treated are usually in need of treatment and/or prevention of a cancer(s). For example, an individual diagnosed with cancer is an animal in need of treatment of cancer, and the particles of the present invention may be administered to such an animal for the purpose of cancer treatment. In those having genetic factors and hence being considered to be at a high risk of developing cancer or those in which cancer has been treated, prevention of cancer development or recurrence is strongly demanded, and the particles of the present invention may be administered to such animals for the purpose of cancer prevention.

The therapeutic and/or prophylactic agent for a cancer(s) according to the present invention comprises the above-described amino acid-containing particles as an effective ingredient. The agent may consist only of the particles, or the particles may be combined with known carriers such as excipient, diluent etc. to prepare the agent in a dosage form suitable for the administration mode.

Examples of the administration method of the therapeutic and/or prophylactic agent include parenteral administration such as subcutaneous, intramuscular, intraperitoneal, intraarterial, intravenous and intrarectal administration as well as oral administration. The agent may be administered systemically, or locally to the tumor and the perilesional sites. Specifically, for example, the agent may be prepared by suspending the antibiotic-containing particles in physiological buffered saline to be administered parenterally by injection or the like, or the agent may be prepared as a capsule or syrup to be administered orally, but the administration method is not restricted thereto.

The dose may be appropriately selected depending on the tumor size, symptoms and the like. Although not restricted, the dose per administration for an adult may be usually about 10 mg to 200 g, particularly about 100 mg to 50 g in terms of the amount of the particles.

### EXAMPLES

The present invention will now be described more concretely by way of an example thereof. However, the present invention is not restricted to the example below.

### 1. Production of Amino Acid-Containing Nanoparticles

Using basic amino acids (Lys, His, Arg), an acidic amino acid (Asp) and neutral amino acids (Gly, Ala, Val, Leu, Try, Phe, Ser, Thr, Met), amino acid-containing cyanoacrylate polymer particles were produced. Dextran 70K was used as a polymerization initiator/stabilizer.

In 10 mL of hydrochloric acid (pH 2), 20 mg of amino acid and 100 mg of dextran 70K were dissolved. The normality of hydrochloric acid used was 0.05 N for basic amino acids, 0.01 N for the basic amino acid hydrochlorides, and 0.01 N for acidic and neutral amino acids. To the solution, 100 µL of nBCA was added under stirring, and the stirring was continued for 120 minutes to proceed polymerization reaction. Aqueous NaOH solution was added dropwise thereto to neutralize the reaction solution (pH7.8), and the resulting solution was stirred for another 30 minutes. The reaction solution was filtered by centrifugal filtration with a Centriprep (YM-10) filter (MILLIPORE) at 3500 rpm/15 min. Distilled water was added to the liquid which did not pass through the filter, and centrifugal filtration was again performed to wash the polymerized particles. This centrifugal washing operation was repeated 5 times in total to obtain particles containing each amino acid. The average particle diameter and the Zeta potential of the obtained particles were measured with a commercially available Zetasizer (manufactured by Malvern Inst.UK). The results are shown in Table 1 below.

**Table 1**

| | Average particle diameter (nm) | Zeta potential (mV) |
|---|---|---|
| Basic amino acid-nanoparticles | | |
| (1) 0.05N D70-Lys-NP | 264 ±45 | -12.1 |
| (2) 0.01N D70-His-NP | 259 ±48 | -23.7 |
| (3) 0.01N D70-Arg-NP | 494 ±90 | -1.07 |

| Acidic amino acid-nanoparticles | | |
|---|---|---|
| (4) 0.01N D70-Asp-NP | 136 ±24 | -18.8 |

| Neutral amino acid-nanoparticles | | |
|---|---|---|
| (5) 0.01 ND70-Gly-NP | 159 ±25 | -26.4 |
| (6) 0.01N D70-Ala-NP | 193 ±35 | -9.82 |
| (7) 0.01N D70-Val-NP | 178 ±28 | -20.9 |
| (8) 0.01N D70-Leu-NP | 173 ±31 | -22.6 |
| (9) 0.01N D70-Try-NP | 182 ±30 | -21.6 |
| (10) 0.01N D70-Phe-NP | 145 ±20 | -22.9 |
| (11) 0.01N D70-Ser-NP | 184 ±29 | -16.6 |
| (12) 0.01N D70-Thr-NP | 171 ±30 | -21.6 |
| (13) 0.01N D70-Met-NP | 185 ±27 | -20.4 |

| Control (Nanoparticles containing no amino acids) | | |
|---|---|---|
| (14) 0.01N D70-NP | 222 ±33 | -19.7 |

| | | |
|---|---|---|
| Values of average particle diameter indicate "mean ± standard deviation". | | |

### 2. Anticancer Activity of Amino Acid-Containing Nanoparticles (Part 1)

Various cell lines were treated with amino acid-containing particles produced above, and cell proliferation assay was carried out by MTT method to assess the cytotoxic activity of the amino acid-containing particles. The assay was performed using MTTassay kit (Roche). The cell lines used were RAW267.4 (murine monocytic leukemia-derived cells), HeLa (human cervical cancer-derived cells), HEK293.T (human embryonic kidney cancer-derived cells) and EL-4 (murine T-cell lymphoma-derived cells).

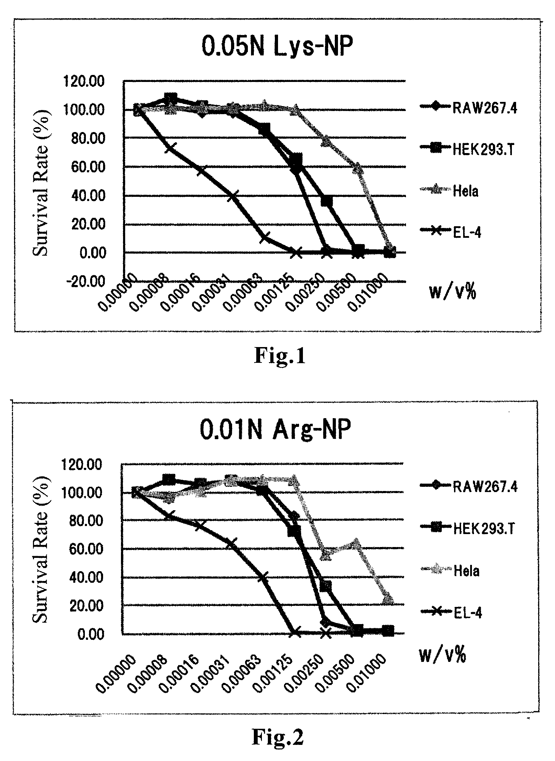

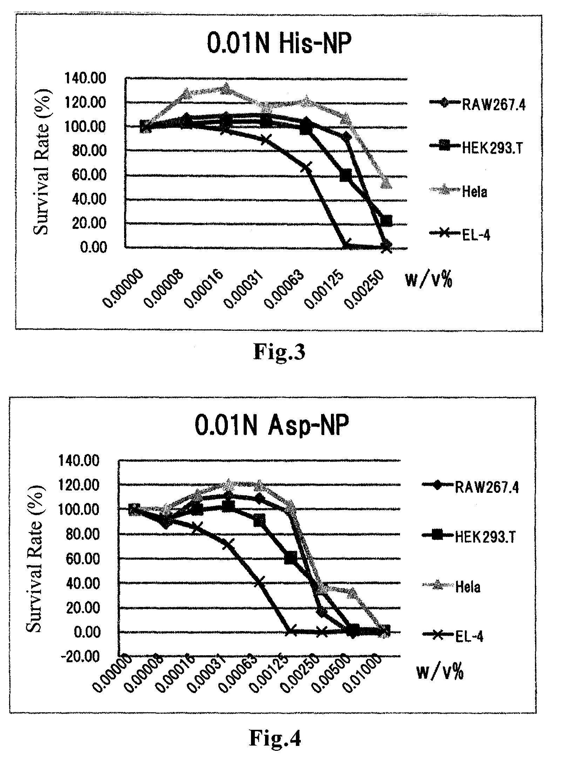

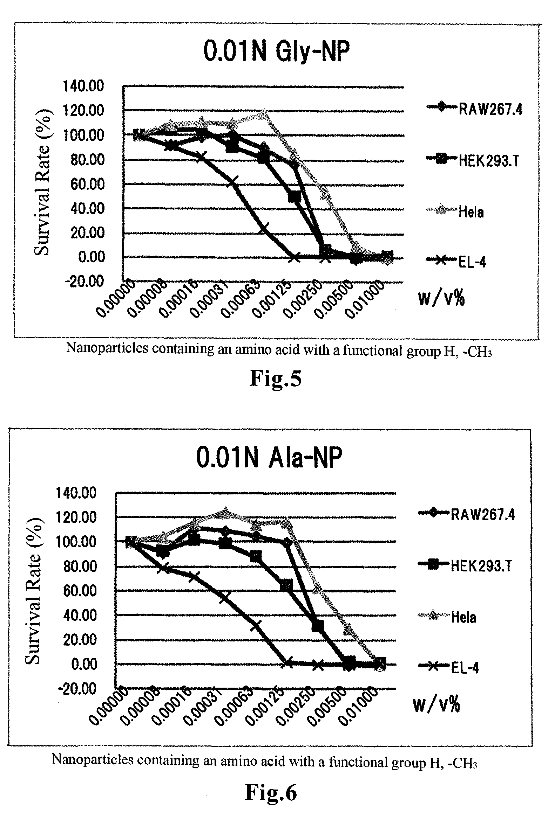

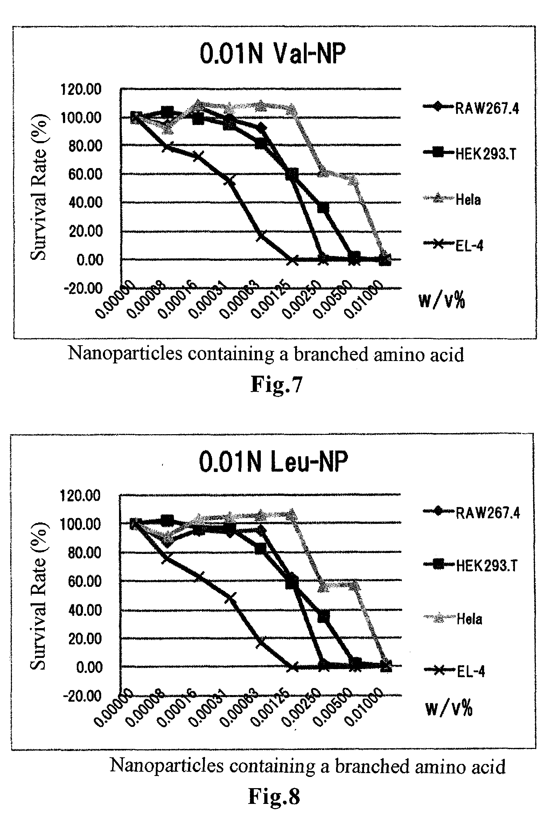

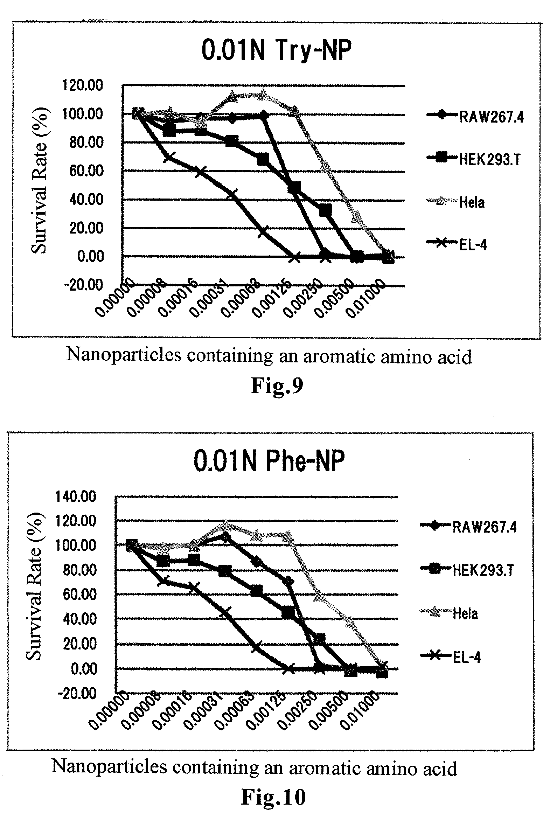

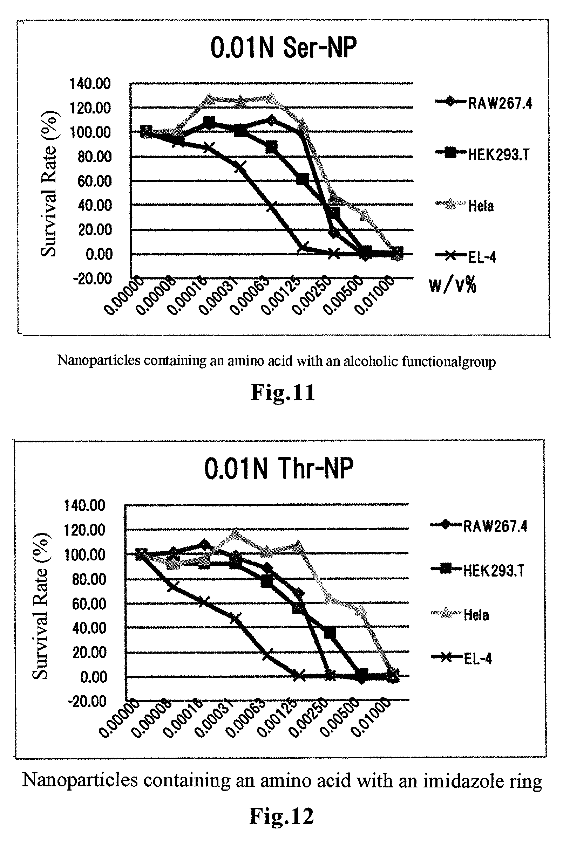

EL-4 cells were adjusted to a cell density of 1 x 10⁵ cells/mL in RPMI-1640 medium to obtain 10 mL of cell suspension. RAW267.4, HeLa and HEK293.T cells were adjusted to a cell density of 1x10⁵ cell/mL in DMEM to obtain 10 mL of cell suspensions, respectively. One-hundred-microliter aliquots of cell suspensions were placed into wells of 96-well cell culture plate (CELLSTAR (registered trademark)), and cultured in a CO₂ incubator at 37°C for 24 hours. The concentration of nanoparticles was adjusted with distilled water, and 10 µl aliquots of each nanoparticle solution were added to the wells, respectively (final concentration of nanoparticles: 0, 0.00008, 0.00016, 0.00031, 0.00063, 0.00125, 0.00250, 0.00500, 0.01000w/v%), followed by further culturing cells in a CO₂ incubator for 24 hours. To each well, 10 µL of MTT labeling reagent was added (final concentration of MTT: 0.5mg/mL/well), and the cells were cultured in CO₂ incubator for 4 hours. One hundred microliters of Solubilization solution was added to each well, and cells were cultured in CO₂ incubator overnight, followed by measuring absorbance at 550 nm. The reference wavelength was set at 700 nm. The results are shown in Figs 1 to 14.

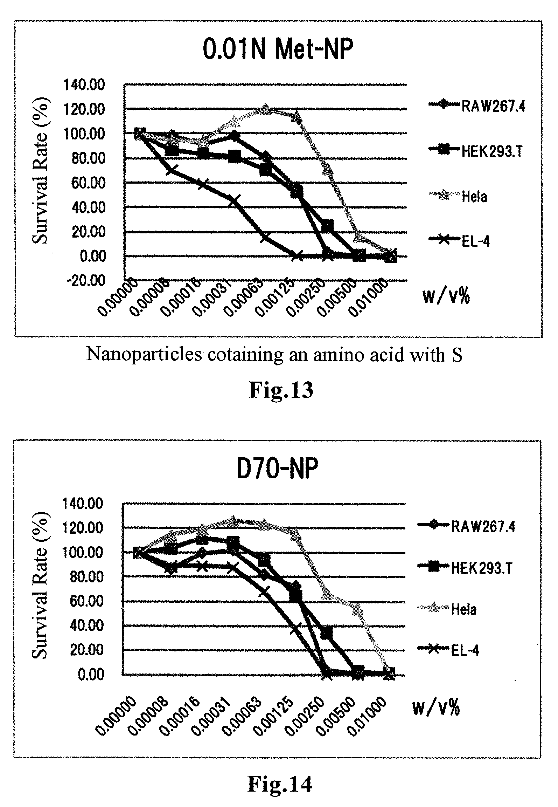

The survival rates (%, relative values obtained by taking the survival rate of particle-untreated cells as 100%) of cells treated with amino acid-containing particles are shown in Figs 1 to 13. The survival rates (%, the same as above) of cells treated with particles containing no amino acid are shown in Fig. 14. In all cases, the survival rate of cells was lowered depending on the particle concentration, indicating that particles exhibit a cytotoxic activity in a concentration-dependent manner. The amino acid-containing particles had an especially high cytotoxic activity to EL-4 cells. The cytotoxic activity to EL-4 cells was about 100-fold higher than the activities to RAW267.4, HeLa and HEK293.T. In particular, lysine-, arginine- and methionine-containing particles had a higher cytotoxic activity to EL-4 compared with particles containing any one of the other amino acids.

### 3. Detection of Apoptosis

During apoptosis, phosphatidylserine (PS) present in the inside of the cell membrane is transferred to the outside of the cell membrane at the early stage, and then the integrity of the cell membrane is disrupted, which finally results in DNA fragmentation. Therefore, using Annexin V having a high affinity for PS and a nuclear staining dye which stains dead cells (such as propidium iodide), cells at the early stage of apoptosis, cells at the late stage of apoptosis and dead cells can be distinguished from each other. In this experiment, Annexin V assay was performed to investigate induction of apoptosis in EL-4 cells treated with arginine-containing nanoparticles.

The experiment was carried out using Annexin V-FITC Apoptosis Detection kit I (BD Biosciences) in accordance with the manufacturer's protocol as follows.
(1) EL-4 cells were washed twice with cold PBS (pH7.4), and then resuspended in 1X Binding buffer at a concentration of up to 1 x 10⁶ cells/mL.
(2) 100 µL of the above-prepared cell suspension (up to 1 x 10⁵ cells) was transferred to 5 mL Falcon tube.
(3) Annexin V reagent (5 µL) or propidium iodide (PI) (2 µL) was added to each tube in the manner shown in Table 2 below.
(4) samples in the tube were gently mixed and incubated for 15 minutes at room temperature in the dark.
(5) 400 µL of 1X Binding buffer was added to each tube. Using the control samples 1 to 5 listed in Table 2 below, compensation was adjusted in accordance with the manufacturer's protocol.
(6) To each tube containing a test sample, arginine-containing particles were added at a final concentration of 100 µg/mL together with 1X Binding buffer, and samples were incubated at 37°C in a CO₂ incubator. The test samples were measured at 2-hour intervals up to 8 hours after the addition of the particles. The measurement conditions were as follows.
Annexin V-FITC:

Absorption maximum 492nm, Emission maximum 520nm
Propidium iodide:
Absorption maxima 370nm and 550nm, Emission range: 560-680nm

| Samples | | Annexin V | PI |
|---|---|---|---|
| 1 | Positive control | - | - |
| 2 | Positive control | o | - |
| 3 | Positive control | - | o |
| 4 | Positive control | o | o |
| 5 | Negative control | o | o |
| 6 | Test sample | o | o |

1-4: Mitomycin or cyclohexamide was used for the apoptosis-induced positive controls.
5: Ultrapure water used for suspending amino acid-containing nanoparticles was used for the apoptosis-uninduced negative control.

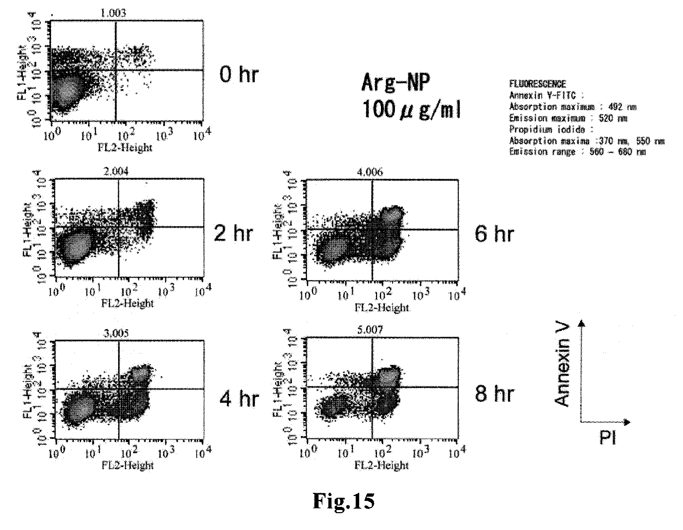

The results of measurement of test samples are shown in Fig. 15. In each graph, the lower left quadrant shows viable cells (Annexin(-), PI(-)); the upper left quadrant shows early apoptotic cells (Annexin(+), PI(-)); the upper right quadrant shows late apoptotic cells (Annexin(+), PI(+)); and the lower right quadrant shows dead cells (Annexin(-), PI(+)). It was confirmed that amino acid-containing particles induce an apoptosis-like reaction in cancer cells.

### 4. Anticancer Activity of Amino Acid-Containing Nanoparticles (Part 2: Hunan B-cell Lymphoma)

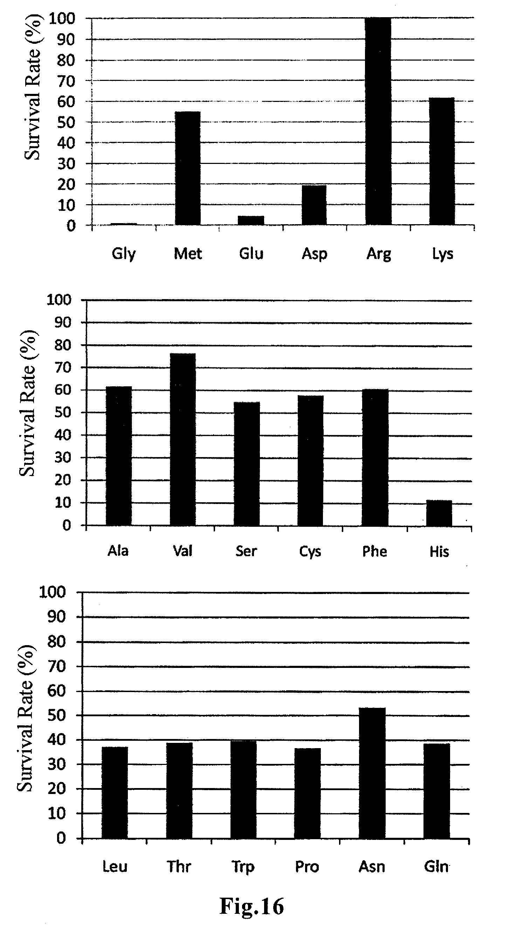

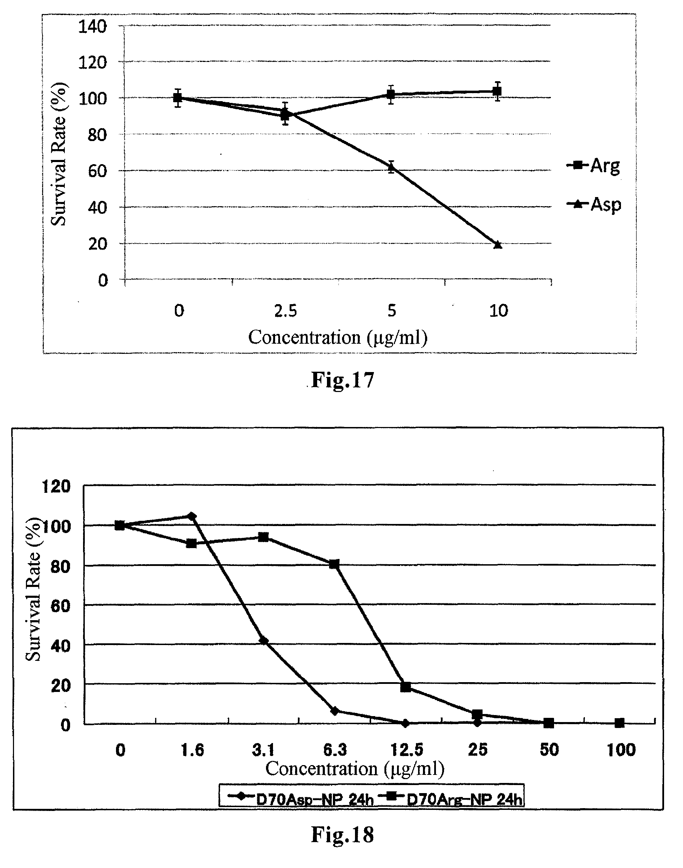

In the same manner as in 2 above, the antiproliferative effect of amino acid-containing particles against human B-cell lymphoma was evaluated by MTT assay using YCUB-2, a known cell line derived from human B-cell lymphoma. YCUB-2 cells were cultured at 37°C under 5% CO₂, and amino acid-containing particles (final concentration 10 µg/ml) were added to the culture. The survival rate of the cells after 48-hour contact with the particles is shown in Fig. 16. The survival rate of the cells after 48-hour treatment with particles at a particle concentration of 0 to 10 µg/ml is shown in Fig. 17.

As shown in Fig. 16, although the antiproliferative effect varied depending on the kind of the amino acid contained in the particles, the cell proliferation was inhibited to about 60% or less by most kinds of the amino acids investigated herein. Arginine-containing particles did not show an antiproliferative effect at a particle concentration of 10 µg/ml. The concentration dependence of the anticancer activity was investigated to obtain the results shown in Fig. 17, which confirm that the amino acid-containing particles also show a higher antiproliferative effect against B-cell lymphoma cells depending on the particle concentration.

### 5. Anticancer Activity of Amino Acid-Containing Nanoparticles (Part 3: Human T-cell Lymphoma)

In the same manner as in 2 above, the antiproliferative effect of amino acid-containing particles against human T-cell lymphoma was evaluated by MTT assay using H9, a known cell line derived from human T-cell lymphoma. H9 cells were cultured at 37°C under 5% CO₂, and amino acid-containing particles were added to the culture at the prescribed concentrations. Cells were contacted with particles for 24 to 72 hours, and the survival rate of the cells was determined.

The concentration dependence of the antiproliferative effect of the particles was examined using the particles at a concentration of 0 to 100 µg/ml with a treatment time of 24 hours. The results are shown in Fig. 18. Although aspartic acid-containing particles had a higher antiproliferative effect than arginine-containing particles, both of the tested particles were confirmed to have a concentration-dependent antiproliferative effect.

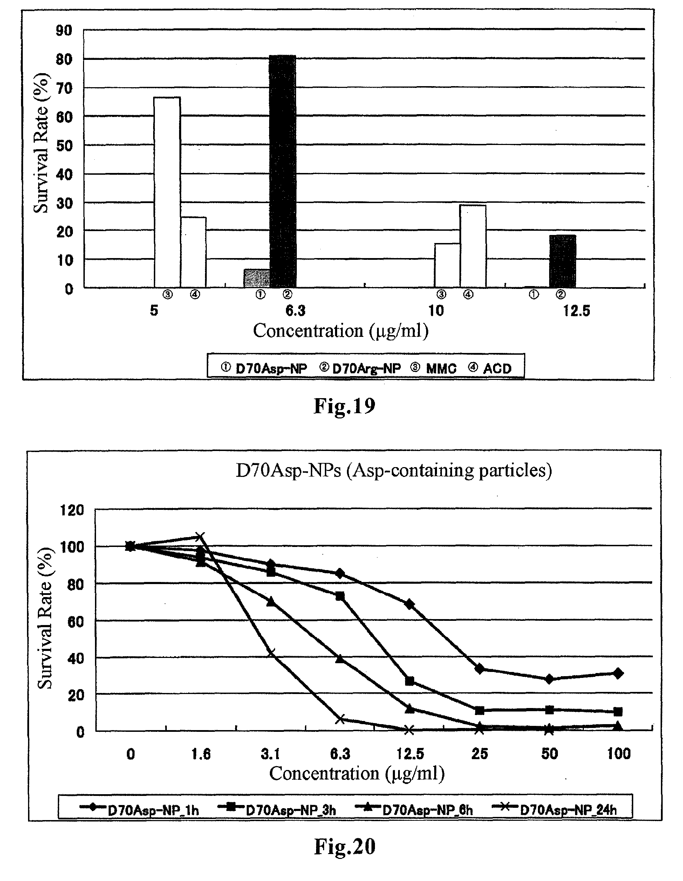

The amino acid-containing particles were compared with the known apoptosis-inducing anticancer agents, mitomycin-C (MMC) and actinomycin-D (ACD), for the antiproliferative effect against H9 cells. H9 cells were treated at the concentrations indicated in the horizontal axis in Fig. 19 for 24 hours, and the survival rate was determined by MTT assay. As a result, as shown in Fig. 19, aspartic acid-containing particles had a remarkably higher antiproliferative effect than the known anticancer agents. Although the antiproliferative effect of arginine-containing particles was lower than that of aspartic acid-containing particles, arginine-containing particles showed the same level of antiproliferative effect as the known anticancer agents when used at a similar concentration thereto.

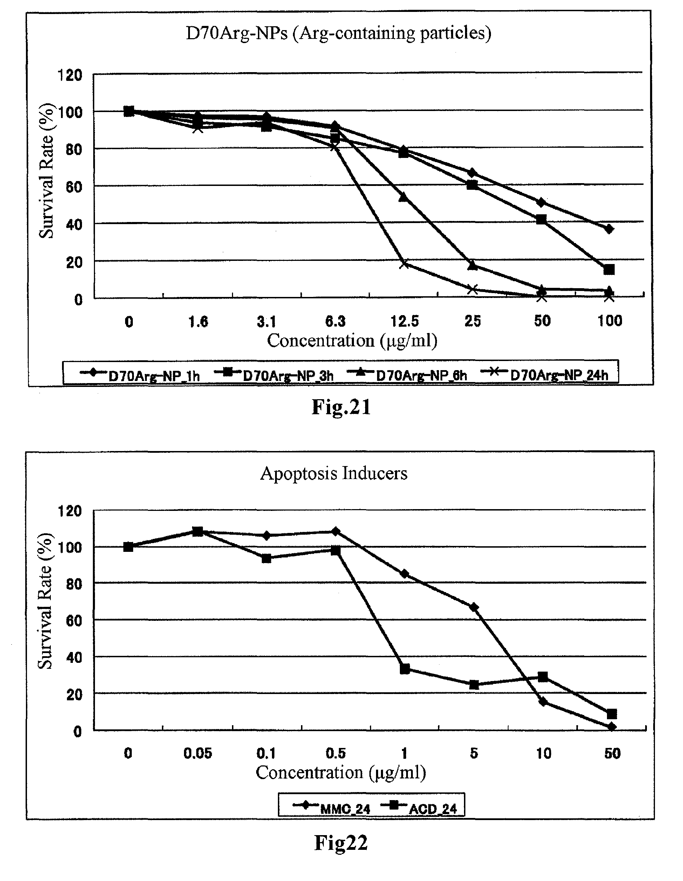

The influence of the treatment concentration and the treatment time was examined in H9 cells using aspartic acid-containing particles. The cells were treated with the particles at a concentration of 0 to 100 µg/ml for 1, 3, 6, or 24 hours. The results are shown in Fig. 20. The antiproliferative effect was elevated depending on not only the treatment concentration but also the treatment time, and a high antiproliferative effect could be observed just by several hours' treatment. The influence of the treatment concentration and the treatment time was also examined using arginine-containing particles in a similar manner. As a result, no differences were observed in the antiproliferation even if cells were treated for a longer time when particles were used at a concentration of 6.3 µg/ml or less. However, the same tendency as aspartic acid-containing particles was observed when particles were used at a higher concentration (Fig. 21). Fig. 22 shows the results of the experiment carried out using the known anticancer agents MMC and ACD in the same manner.

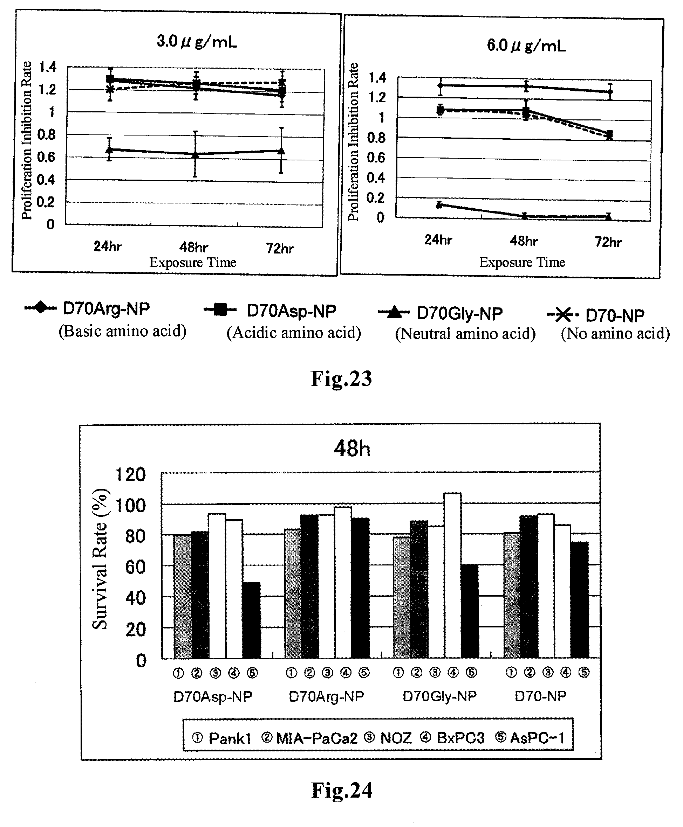

In order to further investigate the anticancer effect of amino acid-containing particles against H9 cells, the particles were classified into those containing a basic amino acid, those containing an acidic amino acid, and those containing a neutral amino acid, and each of the three groups was evaluated for the antiproliferative effect. Arginine was used as a representative basic amino acid; aspartic acid was used as a representative acidic amino acid; and glycine was used as a representative neutral amino acid. Cells were treated with particles at a concentration of 0.75, 1.5, 3.0 or 6.0 µg/ml for 24, 48 or 72 hours. As a result, although any of the particles did not show an anticancer activity at a particle concentration of 0.75 and 1.5 µg/ml (data not shown), the neutral amino acid-containing particles exhibited a strong anticancer activity at a particle concentration of 3.0 and 6.0 µg/ml (Fig. 23). These results suggest that particles containing a neutral amino acid(s) are highly effective against human T-cell lymphoma.

### 6. Anticancer Activity of Amino Acid-Containing Nanoparticles (Part 4: Human Pancreatic Cancer)

In the same manner as 2 above, the antiproliferative effect of amino acid-containing particles against human T-cell lymphoma was evaluated by MTT assay using known cell lines derived from human pancreatic cancer. As pancreatic cancer cells, Pancl, MIA-PaCa2, BxPC3, AsPC-1, and NOZ were used. These cell lines were all derived from human pancreatic cancer, and are known to have differences in the expression profile, response to chemical substances, etc. The cells were cultured at 37°C under 5% CO₂, and amino acid-containing particles were added to each culture at a concentration of 10 µg/ml. Particle treatment was carried out for 48 hours, and the antiproliferative effect was determined by MTT assay.

The results are shown in Fig. 24. The survival rates were calculated taking the survival rate of untreated cells after 48-hour culturing as 100%. Some antiproliferation was observed even in the cells treated with particles containing no amino acid, and the especially remarkable antiproliferative effect was observed in AsPC-1 cells treated with glycine-containing particles or aspartic acid-containing particles, which indicates that amino acid-containing particles can exhibit a therapeutic effect against some of pancreatic cancers.

### 7. In Vivo Toxicity

The *in vivo* toxicity of the amino acid-containing particles was examined in the following two manners.
(1) Amino acid-containing nanoparticles were orally administered to 10 healthy mice at a single dose of 1 g, and the mice were followed up. The mice normally survived without any abnormalities during one month observation after administration.
(2) Each kind of amino acid-containing particle was suspended in physiological saline to prepare 1 mg/ml suspension, and 1 ml of suspension (1 mg of particles) was administered to 5 healthy mice orally, intravenously or intraperitoneally once a week for 4 weeks. The mice were checked for feces, body weight etc. so that no toxicity was found in the mice.

Thus, the amino acid-containing nanoparticles produced above are considered to have a high specificity to cancer cells without injuring normal cells.

## Claims

1. Cyanoacrylate polymer particles which comprise an amino acid(s) and have an average particle diameter of less than 1000 nm.

2. The particles according to claim 1, which are prepared by anionically polymerizing cyanoacrylate monomers in the presence of said monomers, a saccharide(s) and/or polysorbate(s), and an amino acid(s).

3. The particles according to claim 2, wherein said saccharide is at least one saccharide selected from the group consisting of monosaccharides having a hydroxyl group(s) and polysaccharides having a hydroxyl group(s).

4. The particles according to claim 2, wherein said saccharide is dextran.

5. The particles according to any one of claims 1 to 4, wherein said cyanoacrylate is *n*-butyl cyanoacrylate.

6. The particles according to any one of claims 1 to 5, which have an average particle diameter of 20 nm to 600 nm.

7. A therapeutic and/or prophylactic agent for a cancer(s), comprising as an effective ingredient the particles according to any one of claims 1 to 6.

8. The therapeutic and/or prophylactic agent according to claim 7, wherein said cancer is lymphoma.

9. The therapeutic and/or prophylactic agent according to claim 8, wherein said cancer is T-cell lymphoma.

10. The therapeutic and/or prophylactic agent according to claim 9, wherein the amino acid which the particles comprise is at least one selected from the group consisting of glycine, aspartic acid and arginine.

11. The therapeutic and/or prophylactic agent according to claim 8, wherein said cancer is B-cell lymphoma.

12. The therapeutic and/or prophylactic agent according to claim 11, wherein the amino acid which the particles comprise is at least one selected from the group consisting of glycine, methionine, glutamic acid, aspartic acid, lysine, alanine, valine, serine, cysteine, phenylalanine, histidine, leucine, threonine, tryptophan, proline, asparagine, and glutamine.

13. The therapeutic and/or prophylactic agent according to claim 7, wherein said cancer is pancreatic cancer.

14. The therapeutic and/or prophylactic agent according to claim 13, wherein the amino acid which the particles comprise is at least one selected from the group consisting of aspartic acid and glycine.

15. The particles according to any one of claims 1 to 6, for use in treatment and/or prevention of a cancer(s).

16. A method for treatment and/or prevention of a cancer(s), comprising administering an effective amount of the particles according to any one of claims 1 to 6 to an animal in need of such treatment and/or prevention of a cancer(s).
